(19) Europäisches Patentamt
European Patent Office
Office européen des brevets

(11) Publication number: **0 251 126 B1**

(12) **EUROPEAN PATENT SPECIFICATION**

(45) Date of publication of patent specification: **08.01.92**

(51) Int. Cl.$^5$: **C07C 225/16**

(21) Application number: **87108961.1**

(22) Date of filing: **23.06.87**

# (54) 1,7-substituted heptyne-2-ones.

(30) Priority: **27.06.86 US 879397**
**27.04.87 US 42959**

(43) Date of publication of application:
**07.01.88 Bulletin 88/01**

(45) Publication of the grant of the patent:
**08.01.92 Bulletin 92/02**

(84) Designated Contracting States:
**BE CH DE FR GB IT LI LU NL SE**

(56) References cited:

**HELVETICA CHIMICA ACTA, vol. 68, 1985, pages 264-280, Basel, CH; D. SEEBACH et al.: "Tritylketone und Tritylenone. Beiträge zur sterisch erzwungen Michael-Addition und zur diastereoselektiven Aldol-Addition"**

**BULLETIN DE LA SOCIETE CHIMIQUE DE FRANCE, 1959, pages 408-412, Paris, FR; J. COLONGE et al.: "Application du chlorobuténol et chlorobutynol en synthèse (3è mémoire)**

**ANGEWANDTE CHEMIE, vol. 93, no. 6/7, 1981, pages 614-615, Weinheim-Bergstrasse; L. LOCHER et al.: "Michael-Addition von Lithiumacetyliden an Propenyltritylketon"**

(73) Proprietor: **MARION MERRELL DOW INC.**
**9300 Ward Parkway**
**Kansas City Missouri 64114-3321(US)**

(72) Inventor: **Rzeszotarski, Waclaw Janusz**
**407 Buckspur Court**
**Millersville Maryland 21108(US)**
Inventor: **Guzewska, Maria Elzbieta**
**9G Rambling Oaks Way**
**Baltimore Maryland 21228(US)**

(74) Representative: **Abitz, Walter, Dr.-Ing. et al**
**Abitz, Morf, Gritschneder, Freiherr von Wittgenstein Postfach 86 01 09**
**W-8000 München 86(DE)**

**EP 0 251 126 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

a) Field of Invention

This invention relates to novel 1-substituted derivatives of 1-phenyl-1-hydroxy-7-substituted-aminohept-5-yn-2-ones, their pharmaceutically acceptable salts and their use in the treatment of neurogenic bladder disease.

b) State of the Art

Neurogenic bladder disease is defined as a disorder involving loss of control of urination. The major symptoms can be urinary frequency, urinary retention or incontinence.

There are two types of lesions that come under the rubric of neurogenic bladder. The first, upper motoneuron lesion, leads to hypertonia and hyperreflexia of the bladder, a spastic condition, giving rise to symptoms of urinary frequency and incontinence. The second lesion, a lower motoneuron lesion, involves hypotonia and hyporeflexia of the bladder, and in more severe conditions complete distension and atonia of the bladder muscle. Thus, this is a flaccid condition. The major symptoms in this condition are urinary retention, since the voiding reflex has been lost, and incontinence, which occurs when the bladder "leaks", being full to overflowing.

The flaccid or hypotonic bladder is the easier condition to treat. The aim in treatment is to produce a contraction of the bladder, While avoiding contraction of the bladder neck or the urethra. Parasympathomimetic compounds (cholinergic agonists) are commonly employed to stimulate the excitatory muscarinic receptor on the bladder smooth muscle. The most widely used compound in this class is urecholine, a muscarinic receptor agonist. This is given in combination with the alpha-adrenergic antagonist phenoxybenzamine to prevent sympathetic stimulation of the bladder neck muscle. Urecholine is also sometimes given in combination with lioresil, a skeletal muscle receptor antagonist. In general, however, urologists and internists have been moving away from pharmacological treatment of the hypotonic bladder, and prefer to institute the physical maneuver of intermittent catheterization.

The majority of neurogenic bladder patients have the spastic or hypertonic condition, which is usually more difficult to treat. In this instance, the usual aim of the clinician is to attempt to convert the condition of hyperreflexia and hypertonia to hypotonia, thereby treating the primary problem of incontinence. When the condition has been converted to hypotonia it can be straightforwardly managed by intermittent catheterization. There is a significant population of patients who cannot be converted completely from the hypertonic to the hypotonic condition, and who still find that they have to urinate every hour. For those patients, longer term treatment with an anticholinergic drug (muscarinic receptor antagonist) is necessary. The current drug of choice is oxybutynin, which is considered to be better than older anticholinergic treatments such as banthine and probanthine. Although relatively high doses of oxybutynin are in general use (5 mg q.i.d.), tachycardia is not a major side effect of this compound, unlike classical muscarinic receptor antagonists such as atropine. On the other hand, the most frequent side effect of oxybutynin treatment is dry mouth.

Recent advances in the muscarinic receptor field have suggested that oxybutynin is an antagonist with a fair degree of cell activity for the M1-R type of receptor, thereby explaining both the lack of cardiac side effects (heart tissue has predominantly M2-R) and the occurrence of the dry mouth side effect (salivary glands have predominantly an M1-R population.

Although oxybutynin chloride is used in the treatment of neurogenic bladder disorder, it exhibits a relatively short duration of action and, as indicated above, causes dry mouth. The compounds of the present invention contain a methylene group in lieu of the non-carbonyl (i.e. esteratic) oxygen of oxybutynin chloride. These novel compounds exhibit advantages relative to oxybutynin chloride in terms of their efficacy and specificity of action against neurogenic bladder disorder. In particular, the compounds of the invention are longer acting antimuscarinic agents which have spasmolytic action.

Summary of the Invention

The invention provides novel compounds of the formula:

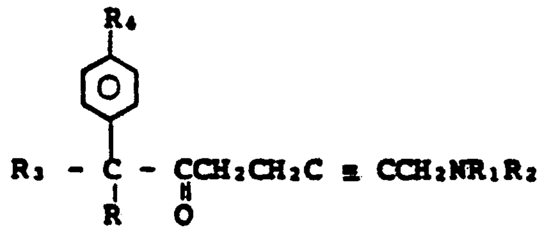

wherein:
R is an alkyl having one to seven carbon atoms, cycloalkyl having three to six carbon atoms, polycycloalkyl of seven to eleven carbon atoms, or substituted or unsubstituted aryl, heterocycloalkyl, or heteroaryl, the substituents being selected from halogen, hydroxy, nitro, methoxy, methyl, trifluoromethyl, $C(O)CH_3$, or amino;
$R_1$ and $R_2$ independently are hydrogen, alkyl having one to three carbon atoms, phenyl alkyl, said alkyl having one to three carbon atoms, or $R_1R_2$ is $-(CH_2)n-$where n is an integer of 4 to 6; and
$R_3$ is hydroxy or fluorine; and
$R_4$ is hydrogen or fluorine;
and the pharmaceutically acceptable salts thereof. The compounds are selective muscarinic acetylcholine receptor (M-AChR) antagonists having particular activity in the treatment of neurogenic bladder disorder.

## Detailed Description of the Invention

The present invention encompasses compounds of the formula:

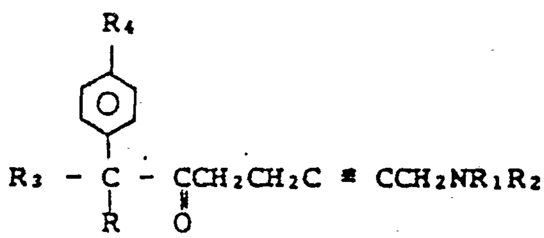

and their pharmaceutically acceptable salts. In the foregoing formula, R is a $C_1$ to $C_7$ alkyl chain; $C_3$ to $C_6$ cycloalkyl such as cyclohexyl and cyclopentyl; aryl including phenyl; polycycloalkyl having seven to eleven carbon atoms; substituted aryl, the substituents being selected from halogen, preferably fluoro, hydroxy, nitro, methoxy, methyl, trifluoromethyl, $C(O)CH_3$ or amino; heterocycloalkyl, such as five and six membered cyclic compounds containing one or two nitrogens preferably bonded to the above noted formula by a methylene group; heteroaryl such as naphthyl and tetrahydronaphthyl; and substituted heteroaryl or heterocyclo wherein the substituents being selected from among those permitted as aryl substituents. Among the heterocyclic substituents suitable in the above formula are pyridyl, including both 3-pyridyl and 4-pyridyl, and five and six membered fully saturated rings having one nitrogen or having two nitrogens para to one another with such ring being bonded to the formula recited above through a methylene group bonded to one of the nitrogens or to a hydrogen para to the single nitrogen of a six-membered ring. Where the heterocyclic compound is substituted, the substituent is preferably bonded to a nitrogen atom and preferably is a methyl or a $-C(O)CH_3$ group. In particular, preferred saturated heterocyclic R substituents include:

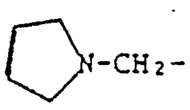, 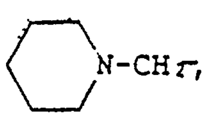, 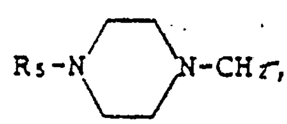, 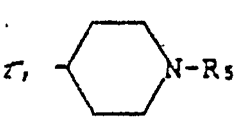

$R_5$ is selected from the substituents permitted as aryl substituents. R is preferably aryl, cycloalkyl or

polycycloalkyl and most preferably phenyl, cycloalkyl of 5 or 6 carbons or adamantyl. When R is phenyl it is preferably para-substituted, most preferably by a fluoro substituent. $R_1$ and $R_2$ are each independently hydrogen, a one to three carbon alkyl, particularly methyl or ethyl, phenyl alkyl, such as phenyl ethyl, or $R_1$ and $R_2$ together with the nitrogen form a heterocyclo group having four to six carbons. $R_1$ is hydroxy or fluorine. $R_4$ is hydrogen or fluorine.

Salts of the compounds of the invention include the acid salts such as the hydrochloride, sulfate, phosphate, nitrate, methanesulfonate and tartrate salts. Other pharmaceutically acceptable salts are also included in the invention.

The preferred compounds of the invention are those in which R is phenyl, cyclohexyl, cyclopropyl, cyclobutyl, cyclopentyl, or 1-adamantyl and $R_1$ and $R_2$ are independently hydrogen, methyl or ethyl. The most preferred compounds are 1-cyclohexyl-1-phenyl-1-hydroxy-7-dimethylaminohept-5-yn-2-one,1-cyclohexyl-1-phenyl-1-hydroxy-7-methylaminohept-5-yn-2-one and 1-cyclohexyl-1-phenyl-1-hydroxy-7-ethylaminohept-5-yn-2-one. Other compounds within the invention include

2-Hydroxy-2-phenyl-8-(N,N-diethylamino)oct-6-yn-3-one oxalate
1-Cyclohexyl-1-hydroxy-1-phenyl-7-diisopropylaminohept-5-yn-2-one hydrate
1-Cyclohexyl-1-hydroxy-1-phenyl-7-ethylamino-hept-5-yn-2-one hemifumarate, hydrate
1-Cyclohexyl-1-hydroxy-1-phenyl-7-(N-methyl-N-ethylamino)hept-5-yn-2-one hydrate
1-Cyclohexyl-1-hydroxy-1-phenyl-7-isopropylaminohept-5-yn-2-onehydrate
1-Cyclohexyl-1-hydroxy-1-phenyl-7-(N-methyl-N-isopropylamino)hept-5-yn-2-one hydrate
1-Cyclohexyl-1-phenyl-7-diethylaminohept-5-yn-2-one oxalate
1-Cyclohexyl-1-hydroxy-1-phenyl-7-t-butylaminohept-5-yn-2-onehydrate
1-Cyclohexyl-1-hydroxy-1-phenyl-7-(N-ethyl-N-isopropylamino)hept-5-yn-2-one
1-Cyclohexyl-1-hydroxy-1-phenyl-7-dimethylaminohept-5-yn-2-one
1-Cyclohexyl-1-hydroxy-1-phenyl-7-(N-methyl-N-phenethylamino)-hept-5-yn-2-one
1-Cyclohexyl-1-hydroxy-1-phenyl-7-pyrrolidinylhept-5-yn-2-one hydrate
1-(6-N,N-Diethylaminohex-4-yn-2-one)-1-hydroxyindan oxalate semihydrate
1-Cyclohexyl-1-hydroxy-1-phenyl-7-methylaminohept-5-yn-2-one hydrate
1-Cyclohexyl-1-fluoro-1-phenyl-7-hept-5-yn-2-one oxalate quarter hydrate
1-Cyclohexyl-1-hydroxy-1-phenyl-7-(N,N-diethyl-N-methylammonio)hept-5-yn-2-one iodide
1-Cyclohexyl-1-hydroxy-1-phenyl-7-(N-methyl-N-benzylamino)hept-5-yn-2-one
1-Cyclopentyl-1-hydroxy-1-phenyl-7-(N-methyl)-N-ethylamino)hept-5-yn-2-one
1-Cyclopropyl-1-hydroxy-1-phenyl-7-diethylaminohept-5-yn-2-one
1-Cyclohexyl-1-hydroxy-1-phenyl-7-aminohept-5-yn-2-one
1-Cyclohexyl-1-hydroxy-1-phenyl-7-di-n-butylaminohept-5-yn-2-one
1-Cyclopentyl-1-hydroxy-1-phenyl-7-dimethylaminohept-5-yn-2-one

The compounds of the invention have a greater affinity toward $M_1$-AChR than $M_2$-AChR and are selective in their pharmacological action. The high selectivity of the compounds of the invention, in particular 1-phenyl or 1-cyclohexyl or 1-cyclopentyl-1-phenyl-1-hydroxy-7-diethylaminohept-5-yn-2-one, has been demonstrated in receptor binding studies, functional assays and isolated organs by their ability to provide a selective antagonism of the $M_1$-AChR.

The compounds may be administered in a variety of pharmaceutical preparations well known to those skilled in the pharmaceutical arts. For parenteral administration, the compounds may be prepared in aqueous injection solutions which may contain antioxidants, buffers, bacteriostats, and other additives commonly employed in such solutions. Extemporaneous injection solutions may be prepared from sterile pills, granules or tablets which may contain diluents, dispersing and surface active agents, binders, and lubricants, as well as the compound of the invention.

In the case of oral administration, fine powders or granules of the compound of the invention may be formulated with diluents and dispersing and surface active agents, and may be prepared in water, a syrup, capsules cachets, a non-aqueous suspension or an emulsion. In dry forms optional binders and lubricants may be present. The compositions may also include flavorants, preservatives, suspending, thickening and emulsifying agents and other pharmaceutically acceptable additives. Granules or tablets for oral administration may be coated.

The compounds are useful in the treatment of patients suffering from neurogenic bladder disorders. The compounds are administered in pharmaceutically effective amounts; that is the amount necessary to produce a anticholinergic effect. Dosing can be determined by correlation of the $M_1$-AChR activity of the compounds of the invention with the known $M_1$-AChR activity of oxybutynin chloride. Typically, the compounds of the invention can be expected to have a longer duration of action thereby permitting a reduced number of doses without loss of activity. Moreover, the acceptable upper dose limits for

compounds of the invention can be higher than for oxybutynin chloride insofar as the compounds of the invention show more selectivity, that is, fewer side effects, than oxybutynin chloride.

The compounds of the invention can be prepared by reaction of an appropriate dithiane and ketone followed by removal of the dithiane protective group. The following examples are illustrative of compounds of the invention and their manufacture:

EXAMPLES

Example I

6-Diethylaminohex-4-yn-1-ol

A mixture of paraformaldehyde (7.14 g, 238 mmol), diethylamine (27.6 ml, 260 mmol) and cupric acetate monohydrate (1 g) in dioxane (40 ml) was heated in an oil bath of 60° C for 1 hour. When the solid material disappeared, pent-4-yn-1-ol (20 g, 238 mmol) was added and the heating at 95° C was continued until greenish suspension had been completely replaced by the thin brown precipitate (approximately 3 hours). After cooling to 20° C, the reaction mixture was poured into 10% aq. KOH (40 ml). The precipitate was filtered off and washed with ether (100 ml). The organic phase was washed with water (5 times 50 ml), dried ($K_2CO_3$) and evaporated to dryness to give crude product. The isolated product was distilled to yield 29.78 g (74%) of 6-diethylaminohex-4-yn-l-ol: bp 92-94° C 0.13 mbar (0.1 mm Hg), IR (neat) 3309 $cm^{-1}$; $^1H$ NMR ($CDCl_3$) $\delta$ 4.1 (S, 1H), 3.6 (t, 2H), 3.3 (m, 2H), 2.7-2.0 (m, 6H), 2.0-1.4 (m, 2H), 1.0 (t, 6H).

6-Diethylaminohex-4-ynal

A solution of dimethyl sulfoxide (25.1 ml, 353 mmol) in $CH_2Cl_2$ (75 ml) was added to the stirred solution of oxalyl chloride (14.75 ml, 169 mmol) in $CH_2Cl_2$ (370 ml) at -60° C. The reaction mixture was stirred for 1 hour and a solution of 6-diethylaminohex-4-yn-1-ol prepared as above (25 g, 147.5 mmol) in $CH_2Cl_2$ (150 ml) was added dropwise; stirring was continued for an additional hour. TEA (103.25 ml, 740 mmol) was added and the reaction mixture was stirred for 90 minutes and then allowed to warm to room temperature. Water (200 ml) was then added and the aqueous layer was reextracted with chloroform (3 times 100 ml). The organic layers were combined, washed with brine and dried ($MgSO_4$). Evaporation of solvent yielded crude product, which was filtered through silica gel and distilled to give 19.26 g (78%) of an aldehyde: bp 68-71° C 0.17 mbar (0.125 mm Hg), IR (neat) 1727.9 $cm^{-1}$; $^1H$ NMR ($CDCl_3$) $\delta$ 9.7 (s, 1H), 3.3 (m, 2H), 2.7-2.2 (m, 8H), 1.0 (t, 6H).

2-(5-Diethylaminopent-3-yn)-1,3-dithiane

A solution of the above prepared 6-diethylaminohex- 4-ynal (10 g, 59.7 mmol) and 1,3-propanedithiol (6 ml, 59.7 mmol) in $CH_2Cl_2$ (150 ml) was stirred for 1 hour and then treated with $BF_3.Et_20$ (8 ml). After 5 hours, the reaction mixture was successively washed three times each with water, 10% aq KOH solution and water. The organic layer was dried ($K_2CO_3$), filtered, and freed of solvent. Chromatography of the residue on silica gel with hexane/ethyl acetate/triethylamine (95:5:3) followed by distillation afforded 4.3 g (28%) of the dithiane: bp 140° C 0.27 mbar (0.2 mm Hg); IR (neat) 907.7 $cm^{-1}$; $^1H$ NMR ($CDCl_3$) $\delta$ 4.1 (t, 1H), 3.4 (m, 2H), 2.8-1.8 (m, 14H), 1.1 (t, 6H), Anal. Calcd. for $C_{13}H_{23}NS_2$: C, 60.65; H, 9.00; N, 5.44; S, 24.91. Found: C, 60.70; H, 9.04; N, 5.42; S, 24.82.

1-Cyclohexyl-1-phenyl-1-hydroxy-7-diethylaminohept-5-yn-2-one bis (1,3-propylene) dithioketal

To the foregoing dithiane (5 g, 19.42 mmol) in 100 ml of dry THF cooled to -70° C was added dropwise 11.4 ml (25 mmol) of 2.2 M n-BuLi. After 45 minutes at this temperature, the reaction mixture was warmed to -20° C and TMEDA (3.76 ml, 25 mmol) was added. After 1 hour at this temperature, the light yellow solution was cooled to -70° C and a solution of cyclohexylphenylketone (3.64 g, 19.4 mmol) in 50 ml of dry THF was introduced. The reaction mixture was stirred for additional 3 hours, then quenched with water, and extracted with ether. The extract was dried ($K_2CO_3$) and freed of solvent. The crude isolated product was chromatographed on silica gel with hexane/ethyl acetate/triethylamine (95:5:3) to yield 6.40 g (74%) of product: IR (neat): 3486.7 $cm^{-1}$; $^1H$ NMR ($CCl_4$) $\delta$ 7.8-7.1 (m, 5H), 3.2 (m, 2H), 2.9-1.3 (m, 26H), 1.0 (t, 6H). Hemioxalate: mp 172-3° C (EtOH); IR (nujol) 1625.1 $cm^{-1}$. Anal. Calcd. for $C_{27}H_{40}NO_3S_2$: C, 66.08; H, 8.22; N, 2.85; S, 13.06. Found: C, 66.18; H, 8.36; N, 2.82; S, 12.95.

1-Cyclohexyl-1-phenyl-1-hydroxy-7-diethylaminohept-5-yn-2-one

Thallium (III) nitrate trihydrate (3.13 g, 6.9 mmol) in methanol (15 ml) was rapidly added to the above dithioketal (3 g, 6.73 mmol) dissolved in MeOH (120 ml) and THF (30 ml). A white precipitate formed immediately and after 5 minutes, $CH_2Cl_2$ (100 ml) was added and the precipitate was filtered. The solvent was removed in vacuum and the residue dissolved in chloroform, washed with water and dried ($MgSO_4$). Evaporation of solvent gave crude 1-cyclohexyl-1-phenyl-1-hydroxy-7-diethylaminohept-5-yn-2-one, which was chromatographed on C-18 silica gel with acetonitrile/MeOH (98:2) to give 340 mg (14.22%) of the desired product: IR (neat) 1709.9 $cm^{-1}$; $^1H$ NMR ($CDCl_3$) $\delta$ 7.6-7.2 (m, 5H), 3.4 (m, 2H), 2.9-2.3 (m, 9H) 2.0-1.0 (m, 17H). Oxalate: mp 112-115°C (EtOH); IR ($CHCl_3$) 1779.8, 1704.8 and 1655.9 $cm^{-1}$. Anal Calcd. for $C_{25}H_{35}NO_6$: C, 67.39; H, 7.92; N, 3.14. Found: C, 67.43; H, 7.95; N, 3.10.

Example II

1-Cyclopentyl-1-phenyl-1-hydroxy-7-diethylaminohept-5-yn-2-one bis (1,3-propylene) dithioketal

To the dithiane prepared in Example I (5 g, 19.42 mmol) in 100 ml of dry THF cooled to -70°C was added dropwise 11.4 ml (25 mmol) of 2.2 M n-BuLi. After 45 minutes at this temperature, the reaction mixture was warmed to -20°C and TMEDA (3.76 ml, 25 mmol) was added. After 1 hour at this temperature, the light yellow solution was cooled to -70°C and a solution of cyclopentyl phenylketone (3.3 ml, 19.42 mmol) in 50 ml of dry THF was introduced. The reaction mixture was stirred for additional 3 hours, then quenched with water, and extracted with ether. The extract was dried ($K_2CO_3$) and the solvent removed in vacuum. The crude isolated product was chromatographed on silica gel with hexane/ethyl acetate/triethylamine (95:5:3) to yield 6.37 g (76%) of product: IR (neat): 3517.5 $cm^{-1}$. $^1H$ NMR ($CCl_4$) $\delta$ 7.7-7.0 (m, 5H), 3.2 (m, 2H), 2.9-1.1 (m, 24H), 1.0 (m, 6H). Oxalate: mp 139-140°C (EtOH); IR (nujol) 1740.8 and 1653.3 $cm^{-1}$; Anal. Calcd. for $C_{27}H_{39}NO_5S_2$: C, 62.16; H, 7.53; N, 2.68; S, 12.29. Found: C, 62.00; H, 7.59; N, 2.63; S, 12.21.

1-Cyclopentyl-1-phenyl-1-hydroxy-7-diethylaminohept-5-yn-2-one

Thallium (III) nitrate trihydrate (3.13 g, 6.9 mmol) in MeOH (15 ml) was rapidly added to the foregoing product (2.9 g, 6.73 mmol) dissolved in MeOH (120 ml) and THF (30 ml). A white precipitate formed immediately and after 5 minutes, $CH_2CL_2$ (100 ml) was added and the precipitate was filtered. The solvents were removed from the filtrate in vacuum and the residue dissolved in chloroform, washed with water and dried ($MgSO_4$). Evaporation of solvent gave crude product, which was chromatographed on C-18 silica gel with acetonitrile/MeOH (98:2) to give 0.77 g (33.62%) of the desired product: IR (neat): 1709.9 $cm^{-1}$; $^1H$ NMR ($CDCl_3$) $\delta$ 7.6-7.1 (m, 5H), 3.3 (m, 2H), 2.8-2.1 (m, 10H), 1.8-1.3 (m, 8H); 1.1 (m, 6H). Oxalate: mp 128-129°C (EtOH); IR ($CHCl_3$): 1776.8, 1704.8 and 1653.3 $cm^{-1}$. Anal. Calcd. for $C_{24}H_{33}NO_6$: C, 66.80; H, 7.70; N, 3.24. Found: C, 66.81; H, 7.74; N, 3.20.

Example III

1,1-Diphenyl-1-hydroxy-7-diethylaminohept-5-yn-2-one bis (1,3-propylene) dithioketal

To the dithiane prepared in Example I (5 g, 19.42 mmol) in 100 ml of dry THF cooled to -70°C was added dropwise 11.4 ml (25 mmol) of 2.2 M n-BuLi. After 45 minutes at this temperature, the reaction mixture was warmed to -20°C and TMEDA (3.76 ml, 25 mmol) was added. After 1 hour at this temperature, the light yellow solution was cooled to -70°C and a solution of benzophenone (3.53 g, 19.4 mmol) in 50 ml of dry THF was introduced. The reaction mixture was stirred for additional 3 hours, then quenched with water, and extracted with ether. The extract was dried ($K_2CO_3$) and freed of solvent. The crude isolated product was chromatographed on silica gel with hexane/ethyl acetate/triethylamine = 95:5:3 to yield 6.16 g (72.13%) of product: IR ($CHCl_3$) 3437.8 $cm^{-1}$; $^1H$ NMR ($CDCl_3$) $\delta$ 7.9-7.0 (m, 10H), 4.2 (s, 1H), 3.2 (m, 2H), 2.6-1.4 (m, 14H), 0.9 (t, 6H).

1,1-Diphenyl-1-hydroxy-7-diethylaminohept-5-yn-2-one

Thallium (III) nitrate trihydrate (3.13 g, 6.9 mmol) in $CH_3OH$ (15 ml) was rapidly added to the dithiane (Example III) 2.95 g, 6.73 mmol) dissolved in $CH_3OH$ (120 ml) and THF (30 ml). A white precipitate formed

immediately and after 5 minutes, $CH_2Cl_2$ (100 ml) was added and the precipitate was filtered. The solvent was removed from the filtrate in vacuum and the residue dissolved in chloroform, washed with water and dried ($MgSO_4$). Evaporation of solvents afforded crude product, which was chromatographed on C-18 silica gel with acetonitrile/MeOH (98:2) to give 0.35 g (15%) of the desired product. IR ($CHCl_3$) 1710.0 $cm^{-1}$; $^1H$ NMR ($CDCl_3$ ) $\delta$ 7.35 (s,10H), 3.3 (m, 2H), 2.8 (t, 2H), 2.5 (m, 6H), 1.0 (t, 6H).

Example IV

6-Diisopropylaminohex-4-yn-l-ol

A mixture of paraformaldehyde (7.14 g, 238 mmol), diisopropylamine (19.1 ml, 262 mmol) and cupric acetate monohydrate (1 g) in dioxane (40 ml) was heated in an oil bath of 60° C for 1 hour. When the solid material disappeared, pent-4-yn-1-ol (20.02 g, 238 mmol) was added and the heating at 95° C was continued until the greenish suspension had been completely replaced by a thin brown precipitate (approximately 3 hours). After cooling to 20° C, the reaction mixture was poured into 10% aq. KOH (40 ml). The precipitate was filtered off and washed with ether (100 ml). The organic phase was washed with water (5 times 50 ml), dried ($K_2CO_3$) and evaporated to dryness to give the crude product. The isolated product was distilled to yield 16.88 g (36%) of 6-diisopropylaminohex-4-yn-l-ol: bp 116-9° C 0.07 mbar (0.05 mm Hg); $^1H$ NMR ($CDCl_3$) $\delta$ 1.08 (d, 12 H), 1.40-1.98 (m, 2H), 2.02-2.60 (m, 2H), 2.83-3.44 (m, 4H), 3.46-3.83 (t, 2H), 4.09 (s, 1H).

6-Diisopropylaminohex-4-ynal

A solution of dimethyl sulfoxide (25.1 ml, 353 mmol) in $CH_2Cl_2$ (75 ml) was added to a stirred solution of oxalyl chloride (14.75 ml, 169 mmol) in $CH_2Cl_2$ (370 ml) at -60° C. The reaction mixture was stirred for 1 hour and a solution of 6-diisopropylaminohex-4-yn-1-ol (29.07 g, 147.5 mmol) in $CH_2Cl_2$ (150 ml) was added and the reaction mixture was stirred for 90 minutes and then allowed to warm to room temperature. Water (200 ml) was then added and the aqueous layer was reextracted with chloroform (3 times 100 ml). The organic layers were combined, washed with brine and dried ($MgSO_4$). Evaporation of the solvent yielded crude product, which was distilled to give 12.96 g of the aldehyde: bp 90-5 0.33 mbar (.25 mm Hg); $^1H$ NMR ($CDCl_3$) $\delta$ 1.05 (d, 12H), 2.00-2.67 (m, 4H), 2.83-3.50 (m, 4H), 9.67 (s, 1H).

2-(5-Diisopropylaminopent-3-yne)-1,3-dithiane

A solution of 6-diisopropylaminohex-4-ynal (11.66 g, 59.7 mmol) and 1.3-propanedithiol (6 ml, 59.7 mmol) in $CH_2Cl_2$ (150 ml) was stirred for 1 hour and then treated with $BF_3$ $Et_20$ (8ml). After 5 hours the reaction was successively washed three times each with water, 10% aq KOH solution and water. The organic layer was dried ($K_2CO_3$), filtered, and freed of solvent. Chromatography of the residue on silica gel with hexane/ethyl acetate/triethylamine (95:5:3) followed by distillation afforded 2.28 g (13%) of the dithiane: bp 150-54° C 0.13 mbar (0.1 mm Hg); $^1H$ NMR ($CDCl_3$)$\delta$ to 0.91-1.50 (d + m, 14H), 1.68-2.67 (m, 6H), 2.73-3.67 (m, 6H) , 4.18 (t, 1H).

1-Cyclohexyl-phenyl-1-hydroxy-7-diisopropylaminohept-5-yne-2-one bis (1,3-propylene) dithioketal

To the dithiane (5.54 g, 19.42 mmol) in 100 ml of dry THF cooled to -70° C 10.0 ml (25 mmol) of 2.5 M n-BuLi was added dropwise. After 45 minutes at this temperature, the reaction mixture was warmed to -20° C and TMEDA (3.76 ml, 25 mmol) was added. After 1 hour at this temperature, the light yellow solution was cooled to -70° C and a solution of cyclohexylphenyl ketone (4.70 g, 25 mmol) in 50 ml of dry THF was introduced. The reaction mixture was stirred for an additional 3 hours, then quenched with water, and extracted with ether. The extract was dried ($K_2CO_3$) and freed of solvent. The crude isolated product was chromatographed on silica gel with hexane/ethyl acetate/triethylamine (95:5:3) to yield 1.64 g (30%) of product.

1-Cyclohexyl-1-phenyl-1-hydroxy-7-diisopropylaminohept-5-yne-2-one

Thallium (III) nitrate trihydrate (3.13 g, 6.9 mmol) in methanol (15 ml) was rapidly added to the foregoing dithioketal, (3.19 g, 6.73 mmol) dissolved in MeOH (120 ml) and THF (30 ml). A white precipitate formed immediately and after 5 minutes, $CH_2Cl_2$ was added and the precipitate was filtered. The solvent was

removed in vacuum and the residue dissolved in chloroform, washed with water and dried ($MgSO_4$). Evaporation of solvent gave crude product, which was chromatographed on C-18 silica gel with acetonitrile/MeOH (98:2) to give the desired product.

Example V

3-Cyclohexyl-3-hydroxy-3-phenylprop-1-yne

Lithium acetylide (47.84g, 0.52mol) was added to 70 ml of dry tetrahyrofuran (THF). The solution was cooled to 0°C and a solution of cyclohexyl phenyl ketone in 100 ml of dry THF was added over a period of 15 minutes with stirring. The solution was allowed to warm to room temperature and stirred for 16 hours. The solution was then cooled to 0°C and 50 ml of a 5H HCl solution was added with. The solution was then warmed to room temperature and 200 ml of water was added. The solution was transferred to a separatory funnel and washed with ether. The ether washes were combined and dried over sodium sulfate. Upon removal of the ether under vacuum and vacuum distillation of the crude product a pale yellow oil was isolated (60.33g, 82.9%). Bp 111-14°C 1.06 mbar (0.8mm). $^1H$ NMR ($CDCl_3$) $\delta$ 7.7-7.2 (m, 5H), 2.6 (s, 1H), 2.3 (s, 1H), 2.1-0.9 (m, 11H). IR (neat) 3433, 3304, 2111, 1448, 1016 $cm^{-1}$.

1-Acetoxy-1-cyclohexyl-1-phenylpropan-2-one

3-Cyclohexyl-3-hydroxy-3-phenylprop-1-yne (21.57g, 0.10mol) was added to 100 ml of glacial acetic acid. While the solution was stirred vigorously mercuric acetate (35.20g, 0.11mol) was added. The solution was stirred at room temperature for 72 hours and then thioacetamide (8.3g, 0.11mol) was added. The solution was stirred an additional 3 hours and 300 ml of ether was added. The reaction mixture was transferred to a separatory funnel and washed with water, a saturated sodium bicarbonate solution, and again with water. The ether layer was dried over sodium sulfate and the ether removed under vacuum to afford the product as a white crystalline solid (18.36g, 66.9%). Mp 81-83°C (from petroleum ether). $^1N$ NMR ($CDCl_3$) $\delta$ 7.3 (bs, 5H), 2.2 (s, 3H), 1.9 (s, 3H), 2.0-0.8 (m, 11H).

1-Cyclohexyl-1-hydroxy-1-phenylpropan-2-one

To a solution of 1-acetoxy-1-cyclohexyl-1-phenylpropan-2-one (16,42g, 0.06mol) in 55 ml of a 90% aqueous methanol solution was added 3.2g of potassium hydroxide. The solution was refluxed for 15 minutes, cooled, and 90 ml of a saturated sodium chloride was added. The reaction mixture was transferred to a separatory funnel and washed with ether. The ether washes were combined, dried over sodium sulfate, and evaporated to dryness under vacuum. The crude product was vacuum distilled to afford a clear oil (10.51g, 75.4%). Bp 125-28°C 0.13 mbar (0.1 mm). $^1H$ NMR ($CDCl_3$) $\delta$ 7.6-7.2 (m, 5H), 4.5 (s, 1H), 2.1 (s, 3H), 2.5-0.9 (m, 11H). IR (neat) 3456, 3057, 1705, 1448, 1358, 1209, 1124 $cm^{-1}$

1-(Trimethylsilyl ether)-1-cyclohexyl-1-phenylpropan-2-one

1-Cyclohexyl-1-hydroxy-1-phenylpropan-2-one is added to dry acetonitrile and the solution is stirred. Bis (trimethylsilyl) acetamide is then added and the reaction mixture is heated at 30°C for 1 hour. The solution is then cooled and evaporated to dryness under vacuum to afford the product as a white crystalline solid after recrystallization.

1-(Trimethylsilyl ether)-1-cyclohexyl-1-phenylhex-5-yn-2-one

Lithium diisopropylamide is dissolved in dry THF and the solution cooled to -78°C. A solution containing 1-(trimethylsilyl ether)-1-cyclohexyl-1-phenylpropan-2-one in dry THF is added dropwise to the stirring solution at -78°C. After the addition is complete the reaction mixture is stirred for 1 hour. Then a solution of propargyl bromide in dry THF is added dropwise to the solution at -78°C. The solution is then warmed to 0°C and stirred for 5 hours. An ether-acetic acid solution (9:1) is slowly added and the resultant mixture warmed to room temperature. Ether is added and the solution transferred to a separatory funnel and washed with water. The ether layer is dried over sodium sulfate and evaporated to dryness to afford the product as a white crystalline solid after recrystallization.

1-Cyclohexyl-1-hydroxyl-1-phenylhex-5-yn-2-one

1-(Trimethylsilyl ether)-1-cyclohexyl-1-phenylhex5-yn-2-one is dissolved in methanol containing 1% concentrated hydrochloric acid at 25 °C. Water is added and the solution transferred to a separatory funnel. The solution is washed with ether and the ether layer dried over sodium sulfate. Upon evaporation of the ether under vacuum and vacuum distillation of the crude product a pale yellow oil is isolated.

1-Cyclohexyl-1-hydroxy-1-phenyl-7-diethylaminohept-5-yn-2-one

A mixture of paraformaldehyde, diethylamine, and cupric acetate monohydrate in dioxane is heated at 60 °C for 1 hour. 1-Cyclohexyl-1-hydroxy-1-phenylhex-5-yn-2-one is then added and the reaction mixture heated at 95 °C for 5 hours. The mixture is cooled and added to a 10% potassium hydroxide solution. The solution is washed with ether and the ether reaction is dried over sodium sulfate and evaporated to dryness under vacuum. The crude product is distilled to give a white crystalline solid as its oxalate salt.

EXAMPLE VI

In Examples VI and VII the test compounds are as follows:

Compound No.

1. 1,1-Diphenyl-1-hydroxy-7-diethylaminohept-5-yn-2-one
2. 1-Cyclohexyl-1-phenyl-1-hydroxy-7-diethylaminohept-5-yn-2-one bis (1,3-propylene)dithioketal hemioxalate
3. 1-Cyclopentyl-1-phenyl-1-hydroxy-7-diethylaminohept-5-yn-2-one bis (1,3-propylene)dithioketal oxalate
4. 1-Cyclohexyl-1-phenyl-1-hydroxy-7-diethylaminohept-5-yn-2-one
5. 1-Cyclopentyl-1-phenyl-1-hydroxy-7-diethylaminohept-5-yn-2-one
6. 2-(5-Diethylaminopent-3-yn)-1,3-dithiane oxalate
7. 2-Hydroxy-2-phenyl-8-(N,N-diethylamino)oct-6-yn-3-one oxalate
8. 2-Pentyn-1-amine; N,N-diethyl-5-[2-phenylmethyl-1,3-diathian-2-yl] oxalate
9. 1-Cyclohexyl-1-hydroxy-1-phenyl-7-diisopropylaminohept-5-yn-2-one hydrate
10. 1-Cyclohexyl-1-hydroxy-phenyl-7-ethylamino-hept-5-yn-2-one hemifumarate, hydrate
11. 1-(6-Diethylaminohex-4-yn-2-one)-1-hydroxy-1,2,3,4-tetrahydronaphthalene N-cyclohexylsulfamate hydrate
12. 1-Cyclohexyl-1-hydroxy-1-phenyl-7-(N-methyl-N-ethylamino)hept-5-yn-2-one hydrate
13. 1-Cyclohexyl-1-hydroxy-1-phenyl-7-isopropylaminohept-5-yn-2-one hydrate
14. 1-Cyclohexyl-1-hydroxy-1-phenyl-7-(N-methyl-N-isopropylamino)hept-5-yn-2-one hydrate
15. 1-Cyclohexyl-1-phenyl-7-diethylaminohept-5-yn-2-one oxalate
16. 1-Cyclohexyl-1-hydroxy-1-phenyl-7-t-butylaminohept-5-yn-2-one hydrate
17. 1-Cyclohexyl-1-hydroxy-1-phenyl-7-(N-ethyl-N-isopropylamino)hept-5-yn-2-one
18. 1-Cyclohexyl-1-hydroxy-1-phenyl-7-dimethylaminohept-5-yn-2-one
19. 1-Cyclohexyl-1-hydroxy-1-phenyl-7-(N-methyl-N-phenethylamino)-hept-5-yn-2-one
20. 1-Cyclohexyl-1-hydroxy-1-phenyl-7-pyrrolidinylhept-5-yn-2-one hydrate
21. 1-(6-N,N-Diethylaminohex-4-yn-2-one)-1-hydroxyindan oxalate semihydrate
22. 1-Cyclohexyl-1-hydroxy-1-phenyl-7-methylaminohept-5-yn-2-one hydrate

Antimuscarinic Protocol

PURPOSE:

This protocol is designed to identify compounds that possess antagonist activity at postsynaptic muscarinic cholinergic receptors on intestinal (ileal) longitudinal smooth muscle and bladder detrusor muscle.

MATERIALS AND METHODS:

Preparation of Ileum for Testing

Male albino guinea-pigs are killed by decapitation or cervical dislocation. The cavity is opened and the small intestine is removed, with about 10 cm of the terminal ileum being discarded. The intestine is placed in a petri dish that contains Tyrodes solution (137mM NaCl, 2.7mM KCl,1.8mM $CaCl_2 \bullet 2H_2O$, 1.1mM $MgCl_2 \bullet 6H_2O$, 0.4mM $NaH_3PO_4$, 11.8mM $NaHCO_3$, 5.6mM dextrose) and cut into 3-4 cm segments. The

segments are preferentially taken from the aboral end of the ileum. Each segment is carefully stretched onto a glass rod 6mm in diameter and the remaining mesenteric tissue is cut away. The longitudinal muscle, with the myenteric plexus attached, is separated from the underlying circular muscle by gently stroking with a cotton-tipped applicator soaked in Tyrodes solution on a tangent away from shallow longitudinal incisions made parallel to the mesenteric attachment. Using gentle traction, and taking care to keep the segment moist throughout the whole procedure, the tissue is stripped from the whole length of the segment (Paton and Zar, J. Physiol. 194:13,1968).

Tissues are suspended with 5-0 silk suture in 10ml water-jacketed glass tissue baths containing Tyrodes solution maintained at 37° C and bubbled with 95% $0_2$/5%$CO_2$. The suture connects each tissue to a isometric force-displacement transducer (Grass or Gould) coupled to a physiograph. Each preparation is suspended under a resting tension of 0.3g and allowed to equilibrate for 36 min. During this period, the baths are emptied and filled every 12 minutes with 10ml warm Tyrodes solution. At the end of this equilibration period, each muscle strip is conditioned by adding 10µm carbachol to the baths. The drug remains in contact with each tissue for 1-2 minutes and then is removed from the bath with 4 rapid rinses of 10ml warm Tyrodes solution. The preparations are allowed to recover for an additional 12 minutes before being used in experiments.

Preparation of Bladder for Testing

Male albino guinea-pigs are killed by decapitation or cervical dislocation. The peritoneal cavity is opened and the bladder is held lightly at its apex, stretched gently, and fat is lifted with fine forceps and dissected away in situ with blunt tipped scissors as close to the surface of the bladder as possible. The tissue is placed in a latex-bottomed Petri dish that contains a modified Krebs solution (133mM NaCl, 1.3mM $NaH_3PO_4$, 16.3mM $NaHCO_3$, 4.7mM KCl, 0.6mM $MgSO_4$•$7H_2O$, 2.5mM $CaCl_2$•$2H_2O$, 7.7mM dextrose) and cut above the neck. The bladder is collapsed into a flat pouch, which is opened by two lateral incisions and unfolded to give a rectangular sheet of tissue approximately 2 cm long and 1 cm wide. The sheet is gently stretched and pinned to the bottom of the Petri dish. Blunt separation of the mucosa, which is visible as a looser superficial pink layer, is started at one end by carefully inserting the blades of micro dissecting scissors between the mucosa and muscle layers and using gentle spreading of the blades, together with steady traction with forceps to tease the two layers apart. Clean removal of the mucosa is usually possible without any fraying or tearing of the underlying muscle. The removal of the mucosa is considered essential for improving oxygen supply to the preparation and for providing better access on both sides of the thin muscle sheet for administered drugs (Ambache and Zar, J. Physiol. 210:671, 1970). The sheet is trimmed, if necessary, and cut londitudinally into four strips.

The strips are tied off with 5-0 silk suture and are then suspended in 10ml water-jacketed glass tissue baths containing the Krebs solution maintained at 35° C and bubbled with 95% $0_2$/5% $CO_2$. The suture connects each tissue to a isometric force-displacement transducer (Grass or Gould) coupled to a physiograph. Each preparation is suspended under a resting tension of 0.5g and allowed to equilibrate for 36 min. During this period, the baths are emptied and filled every 12 min. With 10 ml warm Krebs buffer. At the end of this period, each muscle strip is conditioned by adding 10µM carbachol to the baths. The drug remains in contact with each tissue for 1-2 min and then is removed by four rapid rinses of 10ml warm Krebs buffer. The preparations are allowed to recover for an additional 12 min before being used in experiments.

Preparation of Agonist

Carbachol is dissolved in saline to produce $2x10^{-2}$M stock concentrations. Serial dilutions (1:10) in saline or water are made from the stock solution. Appropriate volumes of these solutions are added cumulatively to the 10 ml tissue baths in order to obtain the desired bath concentrations.

Preparation of Test Compounds

Compounds that are soluble in water or saline are dissolved in these solvents to produce $2x10^{-2}$ or $2x10^{-3}$M stock concentrations. Small amounts of 1N NCl or NaOH, or 95% ethanol may be added for those agents that are not soluble in water or saline alone. Serial dilutions (1:10) in saline or water are made from the stock solution. Compounds that are insoluble in aqueous solvents are dissolved in (DMSO) dimethylsulfoxide to produce $4x10^{-2}$M stock solutions. Serial dilutions (1:10) in water are made from the stock solution. Other solvents may be used when appropriate and will be specifically described in the experimental procedure. Appropriate volumes are then added to the baths in order to obtain the desired bath

concentrations.

EXPERIMENTAL

Appropriate volumes of carbachol solutions are cumulatively added to the 10ml tissue baths to increase the concentration of carbachol in the bath step-by-step without washing out after each single dose. With each concentration step, the tissue contracts isometrically. The next concentration is added only after the preceding contraction has reached a steady value. When the next concentration step does not cause a further increase in contraction, it is assumed that the maximum effect has been obtained. The tissue is then washed with 4 rapid rinses of 10 ml warm Tyrodes solution and allowed to recover for 12 minutes (Van Rossum et al., Arch. int. Pharmacodyn. 143:240, 1963 and 143:299, 1963). Antagonism of carbachol responses in the presence of antagonist are determined by repeating the cumulative addition procedure after the tissue has been exposed to the agonist for 5 minutes.

Three or four different concentrations of antagonist are studied in the same preparations. Responses are expressed relative to the maximum contraction elicited by carbachol in the absence of antagonist. The data is collected via Buxco Data Logger and analyzed by Branch Technology's software package to obtain Kb values for the antagonists.

TABLE I

| Table I. Antimuscarinic Activity in Guinea Pig Bladder and Ileum | | |
|---|---|---|
| Compound | Bladder Kb (nM) | Ileum Kb (nM) |
| Atropine | 1.2 | 2.5 |
| (R,S)-Oxybutynin | 41.6 | 13.2 |
| (S)-Oxybutynin | 269 | 103 |
| (R)-Oxybutynin | 21 | 2.0 |
| 1 | 110 | 350 |
| 2 | enhance | 117 |
| 3 | 1170 | 234 |
| 4 | 144 | 134 |
| 5 | 58 | 42 |
| 6 | N.E. | |
| 7 | 4470 | NC 10μM |
| 8 | 102 | 575 |
| 9 | 275 | 600 |
| 10 | 117 | 90 |
| 11 | 512 | >1000 |
| 12 | 19 | 17 |
| 13 | 102 | 142 |
| 14 | 447 | 432 |
| 15 | 537 | |
| 16 | 288 | >500 |
| 17 | 234 | 111 |
| 18 | 46 | 20 |
| 19 | 282 | 27.5 |
| 20 | 339 | 90 |
| 21 | N.E. | N.E. |
| 22 | 56 | 13.0 |

EXAMPLE VII

DURATION OF ACTION STUDIES WITH in vivo GUINEA PIG CYSTOMETROGRAM MODEL

Introduction:

To assess test compounds with a more intact model system, a method which more closely mimics the natural slow filling of the bladder is used. The slow-filling cystometrogram (CMG) is used clinically to evaluate urinary bladder dysfunction (Ouslander, J., et al., J. Urol., 137:68-71 (1987)).

This clinical CMG is produced by catheterizing the bladder so it may be filled, emptied and refilled with fluid while simultaneously measuring the internal bladder pressure (Pves). Since the rate of filling can alter the response of the reflex system (Coolsaet, B. Neurourol. Urodyn., 4:263-273 (1985)), a slow rate is chosen. This allows the bladder to reach maximum capacity/pressure before sensory systems initiate the normal urge and micturition reflex patterns. We use a modification of this clinical test to determine duration of action of test compounds by repeated filling of the bladder following i.v. administration of the test compound. Peak pressure above the threshold pressure is the primary measure of action which can be inhibited by the test compounds and can be used to determine potency and duration of action in this anesthetized Guinea pig model.

METHODS:

Animal Subjects:

Female Guinea pigs (350-500 g, outbred stock from Charles River Laboratories, origin: Crl: (HA)BR Hartley, raised and kept as V.A.F.) fed Ralston Purina Guinea pig Chow #5025 and tap water ad libidem and kept in small groups (<5) at 21,1 ± 1,1 °C 70° ± 2° F., RH 40-50%, 12 hr light/dark cycle.

Anesthesia:

Urethane (available from Sigma, #U-2500, 1.5 gm/kg i.p.) injection made as 150 mg/ml urethane in water, is given in divided doses of 80% + 20% about 30 min apart due to potential for overdose if delivered in single dose. This maintains a stable level of anesthesia for three (3) or more hours without supplements.

Animal Preparation:

After induction of anesthesia, the animals are shaved about the medial thigh area for later femoral vein exposure and injection. They are placed supine on a heated small animal board to maintain body temperature. A saline-filled urinary catheter made of PE-100 tubing with several lateral holes near the distal tip is placed into the bladder through the urethra. Care is taken to not allow any air into the system and the tip of the catheter is lubricated with a drop of mineral oil. The catheter is connected by a series of 3-way stopcocks to a pressure transducer (Statham/Gould Model p-50), a syringe pump (Harvard Model 2274) and an exit port to empty the bladder after filling. The bladder is emptied by gentle syringe suction at the opened exit port combined with gentle manipulation and pressure on the lower abdomen. It is then rinsed out with 2-3 ml of saline and emptied again. The urethral opening is sealed about the catheter with a purse-string suture (4-0 Dermalon or silk, using a 3/8-circle cutting needle, e.g. P-3). The syringe pump is set to deliver normal saline at room temperature at approximately 0.4 ml/min with the exit port closed and the transducer open to measure the intravesicular bladder pressure during the filling process. The transducer is balanced and calibrated prior to each experiment using a column of water. For most animals, a full-scale range of 0 to 5.0 kPa (equivalent to 0 to 51 cm $H_2O$) is most useful. In some cases, peak contraction pressure will exceed 5 kPa and a rapid switch to a 0 to 10 kPa scale is needed to detect the peak response. A chart recorder (Gould = Model 2400S) records Pves at a chart speed of 0.10 mm/sec.

Drug Preparation:

Test drugs and reference compounds are normally prepared as a solution in normal saline, acidified or made basic as needed to get the compound into solution at the desired concentration. Drugs are delivered at selected mg/kg (calculated as free base) doses i.v. in a volume of approximately 0.5 to 0.8 ml. The selection of the desired dose to test for duration of action is based on preliminary studies where dose-response data are obtained using an escalating dose series of measures on the paradigm below. Normally for an inhibition measure, the ID70-ID80 is selected so that sufficient signal depression is observed but that the signal is not obliterated or masked by the noise of the test system.

Measurement Paradigm:

After "resting" the bladder empty for 5-10 min, the saline infusion is started. The Pves is measured as the filling occurs. For most animals, a point will be reached within 3 to 15 minutes (approximate bladder volume of 1.2 to 6 ml) where a strong contraction occurs during this first control filling maneuver. When this is recognized and the peak pressure has subsided, the bladder is emptied as noted above and a five (5) minute rest period begins. The control fills are repeated with the fixed rest periods at least three times to establish a reproducible baseline control set. A useful average of the last three fills before the drug treatment is the objective of the repeated control fill maneuvers.

(In some animals, a recognizable contraction response is not evident and/or the bladder appears to be not complaint (the pressure rises continuously and rapidly as volume increases). If the contraction response does not appear within 20 min (about 8 ml volume) or the continuously rising Pves exceeds 2.5-3 kPa, empty the bladder and begin the fixed 5 min rest period. Nearly all animals will begin to produce typical control responses after two or three filling maneuvers.)

The following measurements are taken from the chart record:

PvesB = the baseline pressure at the beginning of a fill maneuver
PvesT = the threshold pressure where the oberved contraction response begins
PvesM = the absolute maximum Pves during the contraction response
PvesP = PvesM - PvesT : peak pressure above threshold pressure
Time = Infusion period until PvesT is noted (Volume of the bladder is then the actual delivery rate x time, ignoring any kidney output)

After the last of the desired control fills, the femoral vein is exposed and the test drug is delivered i.v. during the midpoint of the fixed 5 min rest period. The filling maneuvers are then repeated to observe the changes of the recorded parameters over time following the test drug administration. Peak pressure above the threshold (PvesP) is used as the primary measure of inhibition of contraction strength for the duration of action of the test compounds.

Analysis:

Measurements of the listed parameters are tabulated and values for control measures averaged (usually the last three control fills) and used to compare with the post-treatment results. PvesP (and any other parameter) is then transformed to percent change from control:

((Result/Control average)*100) - 100

and plotted against time since drug administration.

Because each animal serves as its own control and there is normal variability between animals, this parameter is then normalized to 100% of maximum response (i.e. maximum depression) so that the maximum depression of control is now defined as 100% of the observed depression. All measures over time then relate to the maximum depression observed.

Using the normalized response, plots are obtained of this measure against time since drug administration. For each animal, the plot is examined and a linear regression line is plotted to determine the slope of recovery of the normalized parameter (slope is thus in terms of %/min). Extreme tail points of data are deleted when appropriate using significance of slope criteria. This selects ponts where the slope begins to significantly differ from zero and includes the previous point at either end of the response recovery curve as appropriate. The regression line is then recalculated. Half-life of the parameter recovery is then taken without regard to the original intercept as:

T-1/2 = 50/slope. (min)

Because half-life measures are known to not be normally distributed, the geometric mean (G.M.) and the range of values are reported for each drug treatment or dose for comparison to other treatments. Ordinary tests of significance between drug treatments can then be made on either the regression parameters of the recovery curves or by using Student's t test on the values calculated for T-1/2.

Results:

Twelve (12) drugs were initially tested in this model to determine T-1/2 values for recovery toward control levels of the PvesP contraction strength parameter (Table II.). The doses selected to test for duration of action were based on the estimates of the ID70-ID80 from prior studies and relate to equipotent doses for this measured parameter. For some of the tested compounds, the doses required to reach an ID70-ID80 were either toxic to Guinea pigs or insoluble. These instances are noted in Table 1 and the initial inhibition for all compounds is tabulated.

All drugs except two showed either the same or shorter half-lives of PvesP depression than oxybutynin.

The two exhibiting significantly longer values were compound 10 (1.78 times longer) and compound 18 (2.65 times longer). Using the F-test for homogeneity of slopes, both these values are significantly different from the slope for okybutynin ($p < 0.00001$). For compound 18, even the extreme lower range of values did not overlap the extreme high range for oxybutynin.

TABLE II

CONTRACTION INHIBITION HALF-LIFE WITH *in vivo* GUINEA PIG CYSTOMETROGRAM (CMG) AT EQUI-EFFECTIVE* INHIBITORY DOSES FOLLOWING BOLUS i.v. INJECTION

| COMPOUND | DOSE (mg/kg) | INITIAL INHIB. (% Change ± SEM from Control) | T-$\frac{1}{2}$ (G.M., range) (min.) | n§ |
|---|---|---|---|---|
| Atropine | 0.03 | -65 ± 9 | 60.5 (40-81) | 4 |
| Oxybutynin | 1.0 | -78 ± 4 | 46.2 (31-69) | 7 |
| 4 | 3.0 | -78 ± 3 | 29.4 (18-38) | 10 |
| 8 | 3.0* | -63 ± 16 | 41.2 (33-51) | 2 |
| 9 | 10.0 | -76 ± 3 | 31.8 (29-35) | 2 |
| 10 | 3.0 | -76 ± 2 | 82.4 (46-120) | 10 |
| 11 | 10.0* | -55 ± 17 | 42.0 (32-56) | 2 |
| 12 | 2.0 | -72 ± 5 | 38.0 (32-51) | 4 |
| 13 | 3.0 | -70 ± 9 | 34.0 (26-46) | 6** |
| 14 | 10.0* | -65 ± 7 | 38.8 (24-56) | 4 |
| 18 | 3.0 | -80 ± 3 | 122.6 (71-193) | 11 |
| 20 | 10.0 | -82 ± 2 | 43.2 (38-51) | 4 |

* Some drugs could not be delivered at a dose high enough for "equipotent" depression of this parameter; for these, the highest dose used is reported.
** Two animals with extreme high values (T-$\frac{1}{2}$ = 495 & 146 min.) were excluded from this set. The G.M. with those values included is 57.0 min.
§ n = number of animals.

## Claims

1. A compound of the formula:

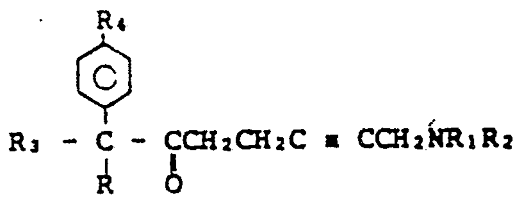

wherein
R is an alkyl having one to seven carbon atoms, cycloalkyl having three to six carbon atoms, polycycloalkyl having seven to eleven carbon atoms, or substituted or unsubstituted aryl, heterocycloalkyl, or heteroaryl, the substituents being selected from halogen, hydroxy, nitro, methoxy, methyl, trifluoromethyl, $C(O)CH_3$, or amino;
$R_1$ and $R_2$ independently are hydrogen, alkyl having one to three carbon atoms, phenyl alkyl, said alkyl having one to three carbon atoms, or $R_1R_2$ is $-(CH_2)n-$ where n is an integer of 4 to 6; and
$R_3$ is hydroxy or fluorine; and
$R_4$ is hydrogen or fluorine;
and the pharmaceutically acceptable salts thereof.

2. The compound of Claim 1 wherein R is substituted or unsubstituted phenyl, naphthyl, tetrahydronaphthyl or pyridyl, the substituents being selected from halogen, hydroxy, nitro, methoxy, methyl, trifluoromethyl, $C(O)CH_3$, or amino.

3. The compound of Claim 1 wherein R is cyclohexyl, cyclopentyl, cyclobutyl, cyclopropyl, adamantane, phenyl or p-fluorophenyl and $R_1$ and $R_2$ are independently hydrogen, methyl or ethyl.

4. The compound of Claim 1 which is 1-cyclohexyl-1-phenyl-1-hydroxy-7-ethylaminohept-5-yn-2-one.

5. The compound of Claim 1 which is 1-cyclohexyl-1-phenyl-1-hydroxy-7-dimethylaminohept-5-yn-2-one.

6. The compound of Claim 1 which is 1-cyclohexyl-1-phenyl-1-hydroxy-7-methylaminohept-5-yn-2-one.

7. A composition for treating neurogenic bladder disorder comprising a pharmaceutically effective amount of a compound of Claim 1.

8. The composition of Claim 7 wherein the compound is 1-cyclohexyl-1-phenyl-1-hydroxy-7-ethylaminohept-5-yn-2-one, 1-cyclohexyl-1-phenyl-1-hydroxy-7-dimethylaminohept-5-yn-2-one or 1-cyclohexyl-1-phenyl-1-hydroxy-7-methylaminohept-5-yn-2-one.

## Revendications

1. Composé de formule :

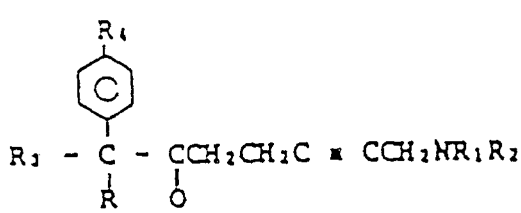

dans laquelle R est un groupe alkyle de 1 à 7 atomes de carbone, cycloalkyle de 3 à 6 atomes de carbone, polycycloalkyle de 7 à 11 atomes de carbone, ou un groupe aryle, hétérocycloalkyle ou hétéroaryle substitué ou non substitué, les substituants étant choisis parmi des atomes d'halogène, tes groupes hydroxy, nitro, méthoxy, méthyle, trifluorométhyle, $C(O)CH_3$ ou amino ;
$R_1$ et $R_2$ étant indépendamment des atomes d'hydrogène, des groupes alkyle de 1 à 3 atomes de carbone, des groupes phénylalkyle, ledit alkyle ayant 1 à 3 atomes de carbone, ou $R_1R_2$ est un groupe $-(CH_2)_n-$ dans lequel n est un entier de 4 à 6 ; et
$R_3$ est un groupe hydroxy ou un atome de fluor ; et
$R_4$ est un atome d'hydrogène ou de fluor ;
et ses sels pharmaceutiquement acceptables.

2. Composé selon la revendication 1, dans lequel R est un groupe phényle, naphthyle, tétrahydronaphtyle ou pyridyle substitué ou non substitué, les substituants étant choisis parmi les atomes d'halogène, les groupes hydroxy, nitro, méthoxy, méthyle, trifluorométhyle, $C(O)CH_3$ ou amino.

3. Composé selon la revendication 1, dans lequel R est un groupe cyclohexyle, cyclopentyle, cyclobutyle, cyclopropyle, adamantane, phényle ou p-fluorophényle, et $R_1$ et $R_2$ sont indépendamment des atomes d'hydrogène ou des groupes méthyle ou éthyle.

4. Composé selon la revendication 1, qui est la 1-cyclohexyl-1-phényl-1-hydroxy-7-éthylamino-5-heptyne-2-one.

5. Composé selon la revendication 1, qui est la 1-cyclohexyl-1-phényl-1-hydroxy-7-diméthylamino-5-heptyne-2-one.

6. Composé selon la revendication 1, qui est la 1-cyclohexyl-1-phényl-1-hydroxy-7-méthylamino-5-heptyne-2-one.

7. Composition pour le traitement des troubles de la vessie neurogène comprenant une quantité pharmaceutiquement efficace d'un composé selon la revendication 1.

8. Composition selon la revendication 7, dans laquelle le composé est la 1-cyclohexyl-1-phényl-1-hydroxy-7-éthylamino-5-heptyne-2-one, la 1-cyclohexyl-1-phényl-1-hydroxy-7-diméthylamino-5-heptyne-2-one ou la 1-cyclohexyl-1-phényl-1-hydroxy-7-méthylamino-5-heptyne-2-one.

## Patentansprüche

1. Verbindung der Formel

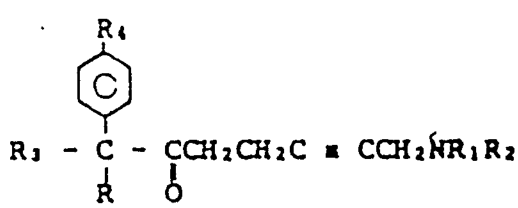

in der
R Alkyl mit 1 bis 7 Kohlenstoffatomen, Cycloalkyl mit 3 bis 6 Kohlenstoffatomen, Polycycloalkyl mit 7 bis 11 Kohlenstoffatomen oder substituiertes oder unsubstituiertes Aryl, Heterocycloalkyl oder Heteroaryl bedeutet, wobei die Substituenten unter Halogen, Hydroxy, Nitro, Methoxy, Methyl, Trifluoromethyl, $C(O)CH_3$ oder Amino ausgewählt sind;
$R_1$ und $R_2$ unabhängig voneinander Wasserstoff, Alkyl mit 1 bis 3 Kohlenstoffatomen oder Phenylalkyl mit 1 bis 3 Kohlenstoffatomen im Alkylrest bedeuten oder $R_1R_2$ die Bedeutung $-(CH_2)_n-$ hat, worin n eine ganze Zahl von 4 bis 6 ist;
$R_3$ Hydroxy oder Fluor bedeutet; und
$R_4$ Wasserstoff oder Fluor bedeutet;

und pharmazeutisch verträgliche Salze davon.

2. Verbindung nach Anspruch 1, worin R substituiertes oder unsubstituiertes Phenyl, Naphthyl, Tetrahydronaphthyl oder Pyridyl bedeutet, wobei die Substituenten unter Halogen, Hydroxy, Nitro, Methoxy, Methyl, Trifluormethyl, $C(O)CH_3$ oder Amino ausgewählt sind.

3. Verbindung nach Anspruch 1, worin R Cyclohexyl, Cyclopentyl, Cyclobutyl, Cyclopropyl, Adamantan, Phenyl oder p-Fluorphenyl bedeutet und $R_1$ und $R_2$ unabhängig voneinander Wasserstoff, Methyl oder Ethyl bedeuten.

4. Verbindung nach Anspruch 1, nämlich 1-Cyclohexyl-1-phenyl-1-hydroxy-7-ethylaminohept-5-in-2-on.

5. Verbindung nach Anspruch 1, nämlich 1-Cyclohexyl-1-phenyl-1-hydroxy-7-dimethylaminohept-5-in-2-on.

6. Verbindung nach Anspruch 1, nämlich 1-Cyclohexyl-1-phenyl-1-hydroxy-7-methylaminohept-5-in-2-on.

7. Zusammensetzung zur Behandlung von neurogenen Blasenstörungen, enthaltend eine pharmazeutisch wirksame Menge einer Verbindung nach Anspruch 1.

8. Zusammensetzung nach Anspruch 7, wobei es sich bei der Verbindung um 1-Cyclohexyl-1-phenyl-1-hydroxy-7-ethylaminohept-5-in-2-on, 1-Cyclohexyl-1-phenyl-1-hydroxy-7-dimethylaminohept-5-in-2-on oder 1-Cyclohexyl-1-phenyl-1-hydroxy-7-methylaminohept-5-in-2-on handelt.